# EUROPEAN PATENT SPECIFICATION

(11) **EP 1 282 725 B1**
(45) Date of publication and mention of the grant of the patent: **02.05.2007**
(21) Application number: 01931464.0
(22) Date of filing: 10.05.2001
(51) Int. Cl.: C12Q 1/48, A61K 45/00, A61K 31/70, A61K 38/45

(54) **METHODS OF MODULATING FUNCTIONS OF POLYPEPTIDE GalNAc TRANSFERASES AND OF SCREENING SUBSTANCES THEREFOR**
METHODEN ZUR MODELIERUNG DIE FUNKTION VON POLYPEPTID GalNAc TRANSFERASEN UND ZURSCREENING SUBSTANZEN DAFÜR
PROCEDES DE MODULATION DE FONCTIONS DE GALNAC-TRANSFERASES POLYPEPTIDIQUES ET DE CRIBLAGE DE SUBSTANCES DE TEST POUR TROUVER DES AGENTS ADAPTES

(30) Priority: 11.05.2000 US 203331 P
(43) Date of publication of application: 12.02.2003
(73) Proprietor: Glycozym ApS, 2970 Horsholm (DK)
(72) Inventor: CLAUSEN, Henrik, DK-2840 Holte (DK); HASSAN, Helle, DK-2000 Frederiksberg (DK); REIS, Celso, Albuquerque, P-4400 Vila Nova de Gaia (PT); BENNETT, Eric, Paul, DK-2800 Lyngby (DK)
(74) Representative: Nyeng, Joergen
(86) International application number: PCT/DK2001/000328
(87) International publication number: WO 2001/085215

(56) References cited:
- WO-A-99/12944
- WO-A-99/64378
- US-A- 5 268 364
- BENOIT SOUDAN ET AL: "Capillary zone electrophoresis and MALDI-mass spectrometry for the monitoring of in vitro O-glycosylation of a threonine/serine-rich MUC5AC hexadecapeptide" JOURNAL OF CHROMATOGRAPHY B, vol. 729, 1999, pages 65-74, XP002902212
- SHIH-FAN KUAN ET AL: "Inhibition of mucin glycosylation by aryl-N-acetyl-alpha-galactosaminides in human colon cancer cells" THE JOURNAL OF BIOLOGICAL CHEMISTRY, vol. 264, no. 32, 1989, pages 19271-19277, XP002902213
- HELLE HASSAN ET AL: "The lectin domain of UDP-N-acetyl-D-galactosamine: polypeptide N-acetylgalactosaminyltransferase-T4 directs its glycopeptide Specificities" THE JOURNAL OF BIOLOGICAL CHEMISTRY, vol. 275, no. 49, 2000, pages 38197-38205, XP002902214
- J C BYRD ET AL: "Inhibition of mucin synthesis by benzyl-alpha-Ga1NAc in KATO III gastric cancer and Caco-2 colon cancer cells" EUROPEAN JOURNAL OF CANCER, vol. 31A, no. 9, 1995, pages 1498-1505, XP002902215
- DATABASE WPI Section Ch, Week 199704 Derwent Publications Ltd., London, GB; Class B03, AN 1995-358575 XP002902216 & JP 07 525558 T (NIPPON SHINYAKU CO LTD), 24 September 1996 (1996-09-24)
- FRED K HAGEN ET AL: "Structure-function analysis of the UDP-N-acetyl-D-galactosamine: polypeptide N-acetylgalactosaminyl-transferase" THE JOURNAL OF BIOLOGICAL CHEMISTRY, vol. 274, no. 10, March 1999 (1999-03), pages 6797-6803, XP002902217

## Description

### TECHNICAL FIELD

The present invention relates generally to the biosynthesis, sorting and secretion of mucins, O-glycosylated glycoproteins, and glycoproteins. In particular, this invention concerns a method of modulating functions of a homologous family of UDP-*N*-acetyl-α-D-galactosamine: polypeptide *N*-acetylgalactosaminyltransferases (GalNAc-transferases), which add *N*-acetylgalactosamine (GalNAc) to the hydroxy group of serine and threonine amino acid residues in peptides and proteins. More particularly, this invention is related to a lectin domain found in the C-terminal region of most GalNAc-transferases, which is distinct from the catalytic domain of the enzymes. Still more particularly, this invention is related to the lectin domains of the GalNAc-transferases, GalNAc-T2, -T3, -T4, and -T7.

Further, this invention concerns a method of screening one or more test substances for the ability to modulate polypeptide GalNAc-transferase enzymatic activity in a cell-free or cell-based assay, in order to find agents which are effective in inhibiting one or more lectin domains of polypeptide GalNAc-transferases and modulating functions mediated by said lectin domains.

### BACKGROUND OF THE INVENTION

Mucin-type O-glycosylation, one of the most abundant forms of protein glycosylation, is found on secreted and cell surface associated glycoproteins of all eukaryotic cells except yeast. Mucin-type O-glycans contribute to a number of important molecular functions, including: direct effects on protein conformation, solubility, and stability; specific receptor functions that regulate cell trafficking and cell-cell interactions; and microbial clearance. Mucin-type O-glycans are synthesised in the Golgi through the sequential addition of saccharide residues, first to hydroxyl groups on serines and threonines of a protein core and subsequently to hydroxyl groups on the growing saccharide chains that extend from the protein core. There is great diversity in the structures created by O-glycosylation (hundreds of potential structures), which are produced by the catalytic activity of hundreds of glycosyltransferase enzymes that are resident in the Golgi complex. Diversity exists at the level of the glycan structure and in positions of attachment of O-glycans to protein backbones. Despite the high degree of potential diversity, it is clear that O-glycosylation is a highly regulated process that shows a high degree of conservation among multicellular organisms.

The factors that regulate the attachment of O-glycans to particular protein sites and their extension into specific structures are poorly understood. Longstanding hypotheses in this area propose that mucin-type O-glycosylation occurs in a stochastic manner where structure of acceptor proteins combined with topology and kinetic properties of resident Golgi glycosyltransferases determine the order and degree of glycosylation (1). This concept does not fully explain the high degree of regulation and specialisation that governs the O-glycosylation process. In particular it is difficult to envision how large mucin molecules with high densities of O-glycans are glycosylated in the Golgi by stochastic mechanisms that also create other sparsely glycosylated proteins.

The first step in mucin-type O-glycosylation is catalysed by one or more members of a large family of UDP-GalNAc: polypeptide *N*-acetylgalactosaminyltransferases (GalNAc-transferases) (EC 2.4.1.41), which transfer GalNAc to serine and threonine acceptor sites (2). To date eight members of the mammalian GalNAc-transferase family have been identified and characterized, and several additional putative members of this gene family have been predicted from analysis of genome databases. The GalNAc-transferase isoforms have different kinetic properties and show differential expression patterns temporally and spatially, suggesting that they have distinct biological functions (2). Sequence analysis of GalNAc-transferases have led to the hypothesis that these enzymes contain two distinct subunits: a central catalytic unit, and a C-terminal unit with sequence similarity to the plant lectin ricin, designated the lectin domain (3-6). Previous experiments involving site-specific mutagenesis of selected conserved residues confirmed that mutations in the catalytic domain eliminated catalytic activity. In contrast, mutations in the lectin domain had no significant effects on catalytic activity of the GalNAc-transferase isoform, GalNAc-T1 (3). Thus, the C-terminal "lectin domain" is believed not to be functional and not to play roles for the enzymatic functions of GalNAc-transferases (3).
Recent evidence demonstrates that some GalNAc-transferases exhibit unique activities with partially GalNAc-glycosylated glycopeptides. The catalytic actions of two GalNAc-transferase isoforms, GalNAc-T4 and -T7, selectively act on glycopeptides corresponding to mucin tandem repeat domains where only some of the clustered potential glycosylation sites have been GalNAc glycosylated by other GalNAc-transferases (7-9). GalNAc-T4 and -T7 recognize different GalNAc-glycosylated peptides and catalyse transfer of GalNAc to acceptor substrate sites in addition to those that were previously utilized. One of the functions of such GalNAc-transferase activities is predicted to represent a control step of the density of O-glycan occupancy in mucins and mucin-like glycoproteins with high density of O-glycosylation. It was hypothesized that such sequential actions of multiple GalNAc-transferase isoforms may be required to complete O-glycan attachments to some mucin peptide sequences allowing for detailed control of density.

One example of this is the glycosylation of the cancer-associated mucin MUC1. MUC1 contains a tandem repeat O-glycosylated region of 20 residues (HGVTSAPDTRPAPGSTAPPA) with five potential O-glycosylation sites. GalNAc-T1, -T2, and -T3 can initiate glycosylation of the MUC1 tandem repeat and incorporate at only three sites (HGVTSAPDTRPAPGSTAPPA, GalNAc attachment sites underlined). GalNAc-T4 is unique in that it is the only GalNAc-transferase isoform identified so far that can complete the O-glycan attachment to all five acceptor sites in the 20 amino acid tandem repeat sequence of the breast cancer associated mucin, MUC1. GalNAc-T4 transfers GalNAc to at least two sites not used by other GalNAc-transferase isoforms on the GalNAc₄TAP24 glycopeptide (TAPPAHGVTSAPDTRPAPGSTAPP, GalNAc attachment sites underlined) (8). An activity such as that exhibited by GalNAc-T4 appears to be required for production of the glycoform of MUC1 expressed by cancer cells where all potential sites are glycosylated (10). Normal MUC1 from lactating mammary glands has approximately 2.6 O-glycans per repeat (11) and MUC1 derived from the cancer cell line T47D has 4.8 O-glycans per repeat (10). The cancer-associated form of MUC1 is therefore associated with higher density of O-glycan occupancy and this is accomplished by a GalNAc-transferase activity identical to or similar to that of GalNAc-T4. The mechanism by which GalNAc-T4 and -T7 recognize and functions with GalNAc-glycosylated glycopeptides is not known.

Glycosylation confers physico-chemical properties including protease resistance, solubility, and stability to proteins (12-14). Glycosylation furthermore confers changes in immunological responses to proteins and glycoproteins. O-glycosylation on mucins and mucin-like glycoproteins protect these molecules found in the extracellular space and body fluids from degradation. Control of O-glycosylation with respect to sites and number (density) of O-glycan attachments to proteins as well as control of the O-glycan structures made at specific sites or in general on glycoproteins, is of interest for several purposes. Diseased cells e.g. cancer cells often dramatically change their O-glycosylation and the altered glycans and glycoproteins may constitute targets for therapeutic and diagnostic measures (15; 16). Mucins functioning in body fluids may have different properties depending on density and structure of O-glycans attached in protection against disease, including infections by micro-organisms. Furthermore, mucins with different glycosylation may change physico-chemical properties including stability and solubility properties that may influence turnover and removal of mucous. A number of lung diseases, e.g. cystic fibrosis, asthma, chronic bronchitis, smokers lungs, are associated with symptomatic mucous accumulation (17-19), and it is likely that the nature and structure of mucins play a role in the pathogenesis of such diseases.

Partial inhibitors of O-glycosylation in cells have been reported. Aryl-*N*-acetyl-α-galactosaminides such as benzyl-, phenyl-, and *p*-nitrophenyl-GalNAc were originally found to inhibit the second step in O-glycosylation, the O-glycan processing step, by inhibiting synthesis of core 1 (Galβ1-3GalNAcα1-R) and more complex structures (20). Benzyl-GalNAc was also found to inhibit sialylation (21; 22). Treatment of cells with benzyl-GalNAc not only inhibits O-glycan processing but also has effects on apical sorting of some O-glycosylated proteins (23-25). The mechanism for this is believed to be through inhibition of sialylation. True inhibitors of O-glycosylation, i.e. inhibitors of the initial O-glycan attachment process governed by polypeptide GalNAc-transferases have not been reported.

Inhibitors of the initiating step in O-glycosylation could completely or selectively block attachment of O-glycans to O-glycosylation sites in proteins. Compounds inhibiting the catalytic function of a selected subset of the polypeptide GalNAc-transferase family may be predicted to only lead to partial inhibition of O-glycosylation capacity of cells. Proteins with no or little O-glycosylation may have entirely different biological properties than their normal glycosylated counterparts. Complete inhibition of O-glycosylation is not desirable because of the many diverse functions of O-glycans, and it is expected to result in cell death. Selective inhibition of O-glycosylation on the other hand is desirable in many cases such as cancer cells producing glycoproteins and mucins with more dense O-glycosylation than normal cells. For example breast cancer cells appear to hyper-glycosylate the cancer-associated cell surface mucin MUC1 compared to glycosylation in normal cells (10). The over-expression of MUC1 and hyperglycosylation found in cancer cells are likely to be important for the pathobiology of cancers. Methods of inhibiting the hyperglycosylation of mucins in cancer cells is desirable. Such inhibitors could be directed to specific isoforms of polypeptide GalNAc-transferases and hence selectively inhibit O-glycan attachments at acceptor sites used by such isoforms. Moreover, such inhibitors could be selectively directed to isoforms functioning as follow-up enzymes with GalNAc-glycopeptide specificities, and inhibitors could also be directed to any other functions polypeptide GalNAc-transferases may have in the secretory pathway and intracellular sorting.

Consequently, there exists a need in the art for methods of inhibiting the functions of polypeptide GalNAc-transferases. Preferable in selectively inhibiting O-glycosylation attachments in glycoproteins and mucins. The present invention meets these needs, and further presents other related advantages.

### SUMMARY OF THE INVENTION

Broadly the present invention provides a method of modulating functions of polypeptide GalNAc-transferases comprising administering an effective amount of an appropriate agent which is effective in modulating functions of one or more polypeptide GalNAc-transferases.

More particularly the present invention provides a method of modulating functions of polypeptide GalNAc-transferases comprising administering an effective amount of an appropriate agent which is effective in inhibiting one or more lectin domains of polypeptide GalNAc-transferases and modulating functions mediated by said lectin domains.

In particular, the invention aims at providing selective inhibitors of the binding properties of the above mentioned lectin domains and selective inhibitors of the effects that these lectin domains exert on the functions of GalNAc-transferases.

The present invention discloses the existence of functional lectin domains associated with the polypeptide GalNAc-transferase isoforms, GalNAc-T2, -T3, -T4, and - T7, and further demonstrates unique functions of these lectin domains in the O-glycosylation process. The GalNAc-transferase isoforms, GalNAc-T4 and -T7, function with partially O-glycosylated peptides as follow-up enzymes. The putative lectin domains found in the C-terminus of these GalNAc-transferases function as genuine lectins with specificity for structures, including but not limited to the following: GalNAc, GalNAcal-R (R represents any aglycon such as benzoyl, phenyl, p-nitrophenyl without limitation), selective GalNAc-peptides, and selective peptides with multiple Ser/Thr O-glycosylation sites. The lectin domains confer unique catalytic properties to the GalNAc-transferases including but not limited to selective GalNAc-glycopeptide substrate specificity, as well as binding properties for peptides and carbohydrates to enhance catalytic properties and other functions related to the O-glycosylation process. In a preferred embodiment, methods of selectively blocking the GalNAc-glycopeptide specificities of such GalNAc-transferase isoforms by GalNAc and GalNAc containing structures are disclosed.

Putative lectin domains with diverse sequences are found on other GalNAc-transferases not displaying apparent GalNAc-glycopeptide substrate specificity. The present invention discloses that the putative lectin domains of GalNAc-T2 and -T3 isoforms also are functional and recognize both carbohydrate and certain peptide sequences. These lectin domains govern additional biological functions for GalNAc-transferases including but not limited to a broader donor substrate specificity. These lectin domains may also function as chaperones in directing mucin-type O-glycosylation, transport and sorting of glycoproteins. In yet a preferred embodiment, methods of blocking the lectin domains of such GalNAc-transferase isoforms by GalNAc and GalNAc containing structures as well as peptides are disclosed.

The present invention also provides a method of screening one or more test substances for the ability to modulate polypeptide GalNAc-transferase enzymatic activity in a cell-free or cell-based assay, which comprises:
(i) contacting a polypeptide GalNAc-transferase, or a cell that recombinantly expresses a polypeptide GalNAc-transferase, with one or more test substances under assay conditions suitable for the detection of said enzymatic activity; and
(ii) measuring whether said enzymatic activity is thereby modulated by one or more of the test substances.

Substances identified as agents which are effective in inhibiting one or more lectin domains of polypeptide GalNAc-transferases may e.g. be selected from the group consisting of naturally or non-naturally occurring carbohydrates, peptides, glycopeptides, glycoconjugates and portions and fragments thereof. They may also be found among nucleic acids as well as small organic or inorganic molecules. They include but are not limited to peptides such as soluble peptides including Ig-tailed fusion peptides, members of random peptide libraries and combinatorial chemistry-derived molecular libraries made of D- and/or L-configuration amino acids, phosphopeptides (including members of random or partially degenerate, directed phosphopeptide libraries), antibodies [e.g. polyclonal, monoclonal, humanized, anti-idiotypic, chimeric, single chain antibodies, fragments, (e.g. Fab, F(ab)2, and Fab expression library fragments, and epitope-binding fragments thereof)], and polypeptides. A substance to be used as an agent according to the invention may be an endogenous physiological compound or it may be a natural or synthetic compound.

Agents in accordance with the present invention are useful for changing the density and sites of O-glycan occupancy in mucins and O-linked glycoproteins. Further uses are in changing Golgi-transport and intracellular sorting events conferred by the lectin domains of GalNAc-transferases. For example, inhibitors of lectin domains of GalNAc-transferases may be useful in manipulating disease-associated 0-glycosylation to augment immunity and to prepare vaccines. Further use may be found in manipulating mucin secretion and O-glycan density in diseases associated with mucous accumulation to decrease secretion and enhance clearance of mucins. Further use may entail modulating O-glycosylation of recombinant glycoproteins by inhibition of polypeptide GalNAc-transferases in host expression cells. These and other aspects of the present invention will become evident upon reference to the following detailed description and drawings.

### BRIEF DESCRIPTION OF THE DRAWINGS

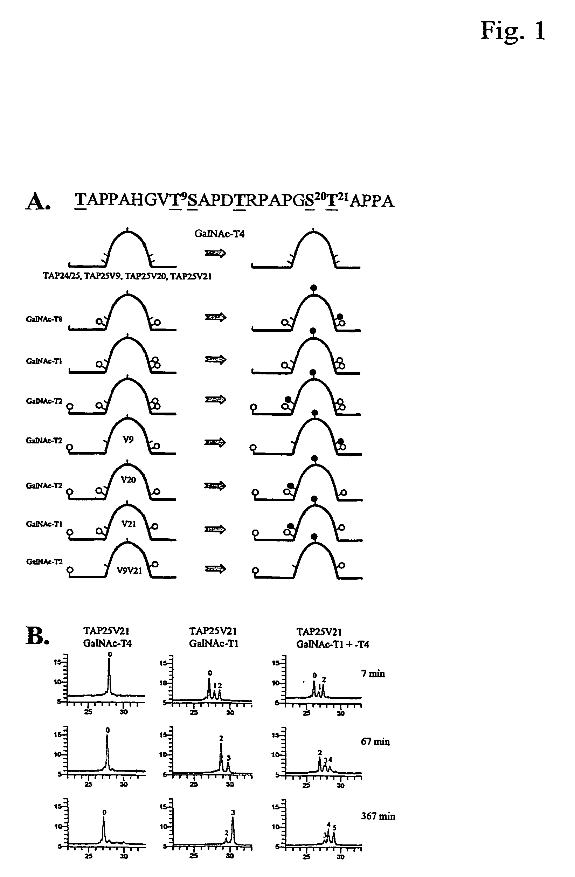

Figure 1 illustrates that the MUC1 glycopeptide specificity of polypeptide GalNAc-T4 is not directed by a specific glycoform. Panel A is a schematic depiction of product development assays monitored by capillary electrophoresis (CE) and/or MALDI-TOF mass spectrometry. Left side illustrates MUC1 tandem repeat peptide glycoforms (open circles indicate attachments of GalNAc) prepared by *in vitro* glycosylation with indicated GalNAc-transferase isoforms. Right side illustrates products (closed circles indicate GalNAc residues added) developed in 6 hours by GalNAc-T4. Glycopeptides were characterized by mass spectrometry. Panel B is an illustration of the reactions with TAP25V21 monitored by capillary electrophoresis, where GalNAc-T1 and -T4 were mixed. Numbers above peaks refer to numbers of moles of GalNAc incorporated into the peptide.

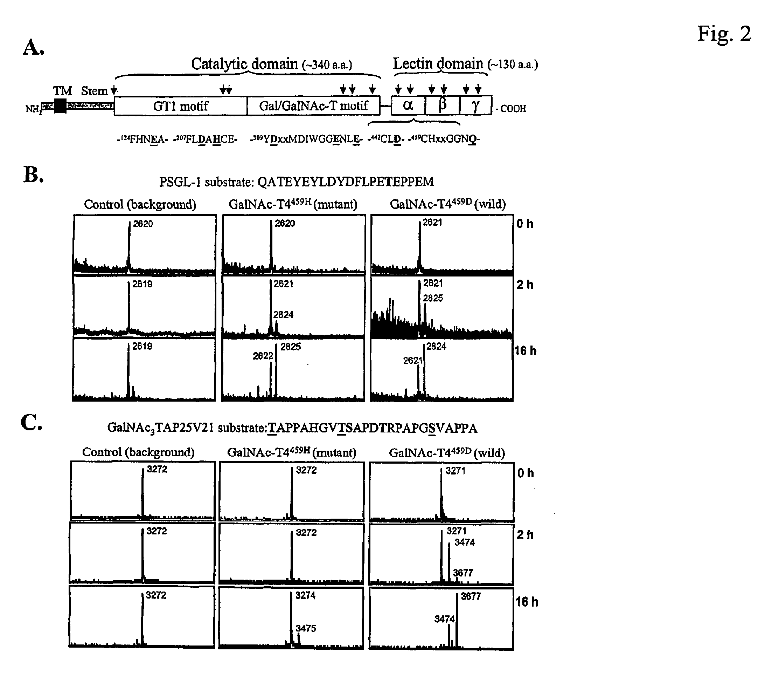

**Figure 2** illustrates that the lectin domain of GalNAc-T4 selectively directs its MUC1 glycopeptide specificity. **Panel A** is a schematic depiction of the domain structure of polypeptide GalNAc-transferases modified from Hagen *et al*. (3). Arrows indicate conserved cysteine residues and the major conserved sequence motifs are shown with numbering according to the sequence of GalNAc-T1. Bold underlined residues in the catalytic domain indicate some residues required for catalysis, whereas the two marked residues in the lectin domain are not essential for catalytic activity of GalNAc-T1 (3). A D459H mutation in the lectin domain of GalNAc-T4 corresponds to the illustrated D444H in GalNAc-T1. **Panel B** is a time-course MALDI-TOF (matrix-assisted-laser-desorption-ionization time-of-flight) analysis of the glycosylation independent activities of wild-type GalNAc-T4^{459D} and the lectin mutant GalNAc-T4^{459H} using the unique substrate for this enzyme isoform derived from PSGL-1 [Thr in bold is the acceptor site (8)]. The control represents co-purified endogenous activity found with irrelevant expression constructs. Wild-type and mutant GalNAc-T4 exhibit identical glycosylation independent activities. **Panel C** is a time-course MALDI-TOF analysis using the unique glycosylation dependent substrate GalNAc₃TAP25V21 (GalNAc attachment sites bold and underlined, and the two available acceptor sites for GalNAc-T4 in bold). The mutant GalNAc-T4 is virtually inactive with the glycopeptide substrate.

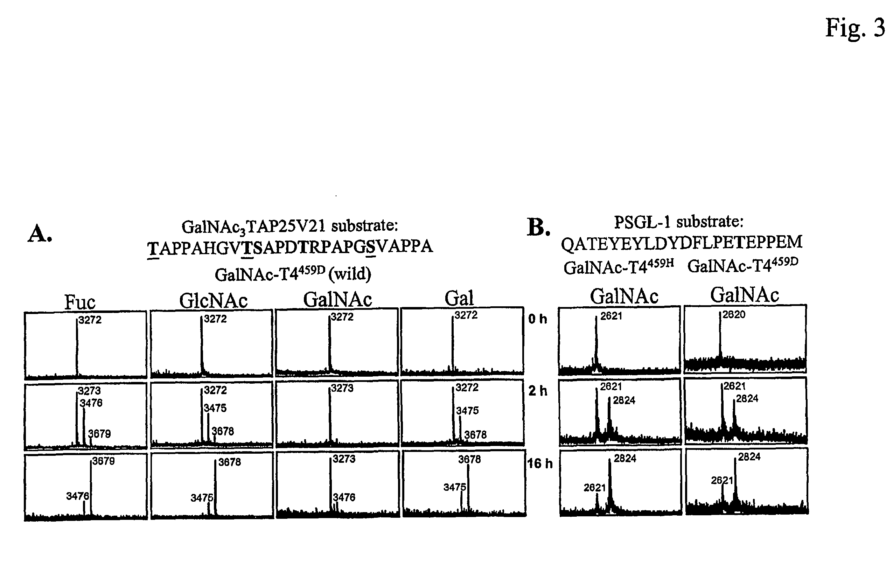

**Figure 3** illustrates that the lectin domain of GalNAc-T4 functions as a lectin and has selective specificity for GalNAc. **Panel A:** Inhibition of the glycosylation dependent function of GalNAc-T4 by free sugars. Time-course MALDI-TOF analysis of GalNAc-T4^{459D} in the presence of 0.23 M free sugars, indicate selective inhibition of activity in the presence of GalNAc. **Panel B**: Time-course MALDI-TOF analysis of the glycosylation independent functions of wild-type and mutant GalNAc-T4, show that GalNAc has no effect on the general catalytic function of the enzyme.

### 1. Definitions

1. "Nucleic acid" or "polynucleotide" as used herein refers to purine- and pyrimidine-containing polymers of any length, either polyribonucleotides or polydeoxyribonucleotides or mixed polyribo-polydeoxyribo nucleotides. This includes single- and double-stranded molecules, i.e., DNA-DNA, DNA-RNA and RNA-RNA hybrids, as well as "protein nucleic acids" (PNA) formed by conjugating bases to an amino acid backbone. This also includes nucleic acids containing modified bases (see below).
2. "Complementary DNA or cDNA" as used herein refers to a DNA molecule or sequence that has been enzymatically synthesised from the sequences present in an mRNA template, or a clone of such a DNA molecule. A "DNA Construct" is a DNA molecule or a clone of such a molecule, either single-or double-stranded, which has been modified to contain segments of DNA that are combined and juxtaposed in a manner that would not otherwise exist in nature. By way of non-limiting example, a cDNA or DNA which has no introns is inserted adjacent to, or within, exogenous DNA sequences.
3. A plasmid or, more generally, a vector, is a DNA construct containing genetic information that may provide for its replication when inserted into a host cell. A plasmid generally contains at least one gene sequence to be expressed in the host cell, as well as sequences that facilitate such gene expression, including promoters and transcription initiation sites. It may be a linear or closed circular molecule.
4. Nucleic acids are "hybridizable" to each other when at least one strand of one nucleic acid can anneal to another nucleic acid under defined stringency conditions. Stringency of hybridization is determined, e.g., by a) the temperature at which hybridization and/or washing is performed, and b) the ionic strength and polarity (e.g., formamide) of the hybridization and washing solutions, as well as other parameters. Hybridization requires that the two nucleic acids contain substantially complementary sequences; depending on the stringency of hybridization, however, mismatches may be tolerated. Typically, hybridization of two sequences at high stringency (such as, for example, in an aqueous solution of 0.5X SSC, at 65°C) requires that the sequences exhibit some high degree of complementarity over their entire sequence. Conditions of intermediate stringency (such as, for example, an aqueous solution of 2X SSC at 65°C) and low stringency (such as, for example, an aqueous solution of 2X SSC at 55°C), require correspondingly less overall complementarily between the hybridising sequences. (1X SSC is 0.15 M NaCl, 0.015 M Na citrate.)
5. An "isolated" nucleic acid or polypeptide as used herein refers to a component that is removed from its original environment (for example, its natural environment if it is naturally occurring). An isolated nucleic acid or polypeptide contains less than about 50%, preferably less than about 75%, and most preferably less than about 90%, of the cellular components with which it was originally associated.
6. A "probe" refers to a nucleic acid that forms a hybrid structure with a sequence in a target region due to complementarily of at least one sequence in the probe with a sequence in the target region.
7. A nucleic acid that is "derived from" a designated sequence refers to a nucleic acid sequence that corresponds to a region of the designated sequence. This encompasses sequences that are homologous or complementary to the sequence, as well as "sequence-conservative variants" and "function-conservative variants". Sequence-conservative variants are those in which a change of one or more nucleotides in a given codon position results in no alteration in the amino acid encoded at that position. Function-conservative variants of polypeptide GalNAc-transferases are those in which a given amino acid residue in the polypeptide has been changed without altering the overall conformation and enzymatic activity (including substrate specificity) of the native polypeptide; these changes include, but are not limited to, replacement of an amino acid with one having similar physico-chemical properties (such as, for example, acidic, basic, hydrophobic, and the like).
8. A "donor substrate" is a molecule recognised by, e.g., a polypeptide GalNAc-transferees and that contributes a *N-*acetylgalactosamine moiety for the transferase reaction. For polypeptide GalNAc-transferases, a donor substrate is UDP-N-acetylgalactosamine or with some GalNAc-transferase isoforms UDP-galactose. An "acceptor substrate" is a molecule, preferably a peptide, protein, glycopeptide, and glycoproteins, that is recognised by, e.g., a polypeptide GalNAc-transferase and that is the target for the modification catalysed by the transferase, i.e., receives the carbohydrate moiety. For polypeptide GalNAc-transferases, acceptor substrates include without limitation peptides, proteins, glycopeptides, and glycoproteins.
9. The term "agonist" refers to a molecule that increases the amount of, or prolongs the duration of, the activity of the polypeptide. The term "antagonist" refers to a molecule which decreases the biological or immunological activity of the polypeptide. Agonists and antagonists may include proteins, nucleic acids, carbohydrates, or any other molecules that associate with a polypeptide GalNAc-transferase.
10. The *N*-acetylgalactosaminyltransferase T4 (GalNAc-T4) gene has been isolated from a human salivary gland library as described previously (8). The sequence of the GalNAc-T4 nucleic acid so isolated and the sequence of the encoded GalNAc-T4 polypeptide have been submitted to GenBank/EBI Data Bank and assigned accession number Y08564.
11. The *N*-acetylgalactosaminyltransferase T7 (GalNAc-T7) gene has been isolated from a human gastric carcinoma cell line MKN45 library as described previously (7). The sequence of the GalNAc-T7 nucleic acid so isolated and the sequence of the encoded GalNAc-T7 polypeptide have been submitted to GenBank/EBI Data Bank and assigned accession number AJ002744.
12. The *N*-acetylgalactosaminyltransferase T2 (GalNAc-T2) gene has been isolated from a human gastric carcinoma cell line MKN45 library as described previously (26). The sequence of the GalNAc-T2 nucleic acid so isolated and the sequence of the encoded GalNAc-T2 polypeptide have been submitted to GenBank/EBI Data Bank and assigned accession number X85019.
13. The *N*-acetylgalactosaminyltransferase T3 (GalNAc-T3) gene has been isolated from a human salivary gland library as described previously (27). The sequence of the GalNAc-T3 nucleic acid so isolated and the sequence of the encoded GalNAc-T3 polypeptide have been submitted to GenBank/EBI Data Bank and assigned accession number X92689.
14. Expression to produce enzymatically-active GalNAc-T4, -T7, -T2, and -T3 can be carried out in any number of conventional expression systems familiar to those skilled in the art. In one embodiment, GalNAc-transferases are expressed in a soluble form which can be recovered from the culture medium. In another embodiment, host cells (e.g. CHO cells) are engineered to express GalNAc-transferases and glycosylate substrates *in vivo* in host cells.

### 2. General aspects of the invention

In accordance with the screening method of the invention, enzymatically active GalNAc-transferases are contacted with an acceptor substrate and an *N*-acetylgalactosamine donor substrate, preferably UDP-*N*-acetylgalactosamine, under conditions for transfer of *N*-acetylgalactosamine from the donor substrate to the acceptor substrate, in the presence of one or more test substances. Glycosylated acceptor substrate is then obtained in a varying degree.

Preferred acceptor substrates are proteins, peptides, glycoproteins, and glycopeptides. Particularly preferred acceptor substrates for GalNAc-T4 are GalNAc-glycosylated glycopeptides from MUC1, MUC2, and MUC5AC tandem repeats or multimers of those molecules. Particularly preferred acceptor substrates for GalNAc-T7 are GalNAc-glycosylated glycopeptides from MUC2 and rat submaxillary gland mucin tandem repeats or multimers of those molecules. Particularly preferred acceptor substrates for GalNAc-T2 are peptides from MUC1, MUC2, MUC5AC and MUC7 tandem repeats or multimers of those molecules. Particularly preferred acceptor substrates for GalNAc-T3 are peptides from MUC1, MUC2, MUC5AC and MUC7 tandem repeats or multimers of those molecules.

Transfer assays for carrying out glycosylation are familiar to those in the art, and are described in the literature cited above and in the examples provided below.

As noted, human GalNAc-T4 demonstrates unique acceptor substrate specificity. GalNAc-T4 has been found to transfer GalNAc to two sites in the MUC1 tandem repeat sequence: Ser in GVTSA and Thr in PDTR using a 24-mer glycopeptide with GalNAc residues attached at sites utilized by GalNAc-T1, -T2 and T3 (TAPPAHGVTSAPDTRPAPGSTAPPA, wherein the GalNAc sites are underlined) (8). In an important aspect of the invention, the action of GalNAc-T4 is dependent on prior GalNAc attachments at least at one site of the five acceptor sites in the MUC1 tandem repeat. In another important aspect of the invention this activity is dependent on a lectin domain constituting approximately 130 amino acid residues in the C-terminal region of GalNAc-T4. In yet another important embodiment of the invention this activity can be blocked by GalNAc and GalNAc containing compounds such as benzyl-GalNAc.

As noted, human GalNAc-T7 demonstrates unique acceptor substrate specificity. GalNAc-T7 has only been found to transfer to acceptor substrates which have previously been partially GalNAc-glycosylated (7; 9). A preferred acceptor substrate is derived from MUC2, MUC5AC and rat submaxillary gland mucin tandem repeats. In an important embodiment of the invention the activity of GalNAc-T7 can be blocked by GalNAc and GalNAc containing compounds such as benzyl-GalNAc, and Gal and Gal containing compounds such as benzyl-Gal and Galβ1-3GalNAcα1-benzyl.

Human GalNAc-T2 demonstrates unique UDP-Gal donor substrate specificity with MUC2 peptide substrate (28). In an important embodiment of the invention the activity of GalNAc-T2 with UDP-Gal can be blocked by GalNAc and GalNAc containing compounds such as benzyl-GalNAc.

Human GalNAc-T3 demonstrates unique UDP-Gal donor substrate specificity with rat submaxillary gland mucin peptide substrate. In an important embodiment of the invention the activity of GalNAc-T3 with UDP-Gal can be blocked by GalNAc and GalNAc containing compounds such as benzyl-GalNAc.

The methods described herein are designed to identify substances and compounds that modulate the biological activity of a polypeptide GalNAc-transferase including substances that interfere with or enhance the activity of a polypeptide GalNAc-transferase.

Agents which modulate a polypeptide GalNAc-transferase can be identified based on their ability to associate with such a transferase. Therefore, the invention also provides a method of identifying agents that associate with a polypeptide GalNAc-transferase. Agents identified using the method of the invention may be isolated, cloned and sequenced using conventional techniques. An agent that associates with a polypeptide GalNAc-transferase may be an agonist or antagonist of the biological or immunological activity of the transferase.

Agents which can associate with a polypeptide GalNAc-transferase may be identified by reacting such GalNAc-transferase with a test substance which potentially associates with a polypeptide GalNAc-transferase under conditions which permit the association, and removing and/or detecting the associated GalNAc-transferase and substance. The substance-transferase complex, free substance, or non-complexed transferase may be assayed. Conditions which permit the formation of substance-transferase complexes may be selected having regard to factors such as the nature and amounts of the substance and the transferase.

The substance-transferase complex, free substance or non-complexed transferase may be isolated by conventional isolation techniques, for example, salting out, chromatography, electrophoresis, gel filtration, fractionation, absorption, polyacrylamide gel electrophoresis, agglutination, or combinations thereof. To facilitate the assay of the components, a labelled antibody against the transferase or the substance or a labelled transferase or a labelled substance may be utilized. The antibodies, transferases, or test substances may be labelled with a detectable substance as described above.

A polypeptide GalNAc-transferase, or a test substance used in the method of the invention may be insolubilized. For example, a transferase, or a test substance may be bound to a suitable carrier such as agarose, cellulose, dextran, Sephadex, Sepharose, carboxymethyl cellulose polystyrene, filter paper, ion-exchange resin, plastic film, plastic tube, glass beads, polyamine-methyl vinylether-maleic acid copolymer, amino acid copolymer, ethylene-maleic acid copolymer, nylon, silk, etc. The carrier may be in the shape of, for example, a tube, test plate, beads, disc, sphere etc. The insolubilized transferase or substance may be prepared by reacting the material with a suitable insoluble carrier using known chemical or physical methods, for example, cyanogen bromide coupling.

The invention also contemplates a method for evaluating an agent for its ability to modulate the biological activity of a polypeptide GalNAc-transferase by assaying for an agonist or antagonist (i.e. enhancer or inhibitor) of the association of the transferase with a substance that interacts with the polypeptide (e.g. donor or acceptor substrates or parts thereof). The basic method for evaluating whether an agent is an agonist or antagonist of the association of a polypeptide GalNAc-transferase and a substance that associates with the transferase is to prepare a reaction mixture containing the transferase and the substance under conditions which permit the formation of substance-transferase complexes, in the presence of a test agent. The test agent may be initially added to the mixture, or may be added subsequent to the addition of the transferase and substance. Control reaction mixtures without the test agent or with a placebo are also prepared. The formation of complexes is detected and the formation of complexes in the control reaction, but not in the reaction mixture, indicates that the test agent interferes with the interaction of the transferase and substance. The reactions may be carried out in the liquid phase or the transferase, substance, or test agent may be immobilized as described herein.

It will be understood that the agonists and antagonists, i.e. enhancers and inhibitors, that can be assayed using the methods of the invention may act on one or more of the interaction sites on the transferase or substance including agonist binding sites, competitive antagonist binding sites, non-competitive antagonist binding sites or allosteric sites.

Preferred agents for inhibition of GalNAc-transferase lectins are inactive as acceptor substrates for glycosyltransferases. In particular, the following glycosyltransferase activities are inactive with the preferred inhibitory compounds: core 1 UDP-Gal:GalNAc-peptide β1,3-galactosyltransferases, CMP-NeuAc:GalNAc-peptide α2,6-sialyltransferases, and UDP-GlcNAc:β1,3N-acetylglucosaminyltransferases involved in 0-glycosylation. Examples of such inhibitory compounds are GalNAcα1-O-benzoyl with substitution of hydroxyl groups at C3 and/or C6 by methyl or acetyl groups to block acceptor sites.

The invention also makes it possible to screen for antagonists that inhibit the effects of an agonist of the interaction of a polypeptide GalNAc-transferase with a substance capable of associating with the transferase. Thus, the invention may be used to assay for an agent that competes for the same interacting site of a polypeptide GalNAc-transferase.

Agents which are effective in modulating a polypeptide GalNAc-transferase can be identified based on their ability to interfere with or enhance the activity of the transferase. Therefore, the invention provides a method for evaluating a test substance for its ability to modulate the activity of a polypeptide GalNAc-transferase comprising
(a) reacting an acceptor substrate and a donor substrate for a GalNAc-transferase polypeptide in the presence of a test substance;
(b) measuring the amount of donor substrate transferred to acceptor substrate, and
(c) carrying out steps (a) and (b) in the absence of the test substance to determine if the substance interferes with or enhances transfer of the sugar donor to the acceptor by the polypeptide GalNAc-transferase.

Suitable acceptor substrates for use in the methods of the invention are polypeptides, glycopolypeptides, or glycoproteins which are either synthetic or naturally occurring structures. Acceptors will generally comprise the hydroxyamino acids serine and/or threonine. The donor substrate may be a nucleotide sugar, dolichol-phosphate-sugar or dolichol-pyrophosphate-oligosaccharide, for example, uridine diphospho-N-acetylgalactosamine (UDP-GalNAc), uridine diphospho-galactose (UDP-Gal), or derivatives or analogs thereof. The GalNAc-transferase polypeptide may be obtained from natural sources or produced using recombinant methods as described and referenced herein.

The acceptor or donor substrates may be labelled with a detectable substance as described herein, and the interaction of the polypeptide of the invention with the acceptor and donor will give rise to a detectable change. The detectable change may be colorimetric, photometric, radiometric, potentiometric, etc. The GalNAc-transferase polypeptide is reacted with the acceptor and donor substrates at a pH and temperature effective for the polypeptide to transfer the donor to the acceptor, and where one of the components is labeled, to produce a detectable change. It is preferred to use a buffer with the acceptor and donor to maintain the pH within the pH range effective for the polypeptides. The buffer, acceptor and donor may be used as an assay composition. Other compounds such as EDTA and detergents may be added to the assay composition.

The reagents suitable for applying the methods of the invention to evaluate agents that modulate a polypeptide GalNAc-transferase may be packaged into convenient kits providing the necessary materials packaged into suitable containers. The kits may also include suitable supports useful in performing the methods of the invention.

Agents that modulate a polypeptide GalNAc-transferase can also be identified by treating immortalized cells which express the transferase with a test substance, and comparing the O-glycosylation performed of the cells with the O-glycosylation of the cells in the absence of the test substance and/or with immortalized cells which do not express the transferase. Examples of immortalized cells that can be used include human breast cancer cell lines such as T47D, which express the polypeptide GalNAc-T4 isoform and produce hyperglycosylated MUC1. In the absence of an inhibitor the cells will produce MUC1 with near complete O-glycosylation of all five potential acceptor sites in the MUC1 tandem repeat sequence. A substance that reduces the density of O-glycosylation in the MUC1 tandem repeat sequence may be considered an inhibitor.

The agents identified by the methods described herein, may be used for modulating the biological activity of a polypeptide GalNAc-transferase, and they may be used in the treatment of conditions mediated by a polypeptide GalNAc-transferase. In particular, they may be used to alter density of O-glycosylation on glycoproteins and mucins produced by cells, and other functions governed by the polypeptide GalNAc-transferases in transport and secretion of glycoproteins and mucins.

Therefore, the present invention may be useful for treatment of various disorders associated with aberrant O-glycosylation and/or mucin production in mammals, preferably humans. Such disorders include the following: tumors and cancers, lung diseases associated with mucous accumulation such as asthma, chronic bronchitis, smoker's lung, cystic fibrosis, diseases of exocrine glands associated with increased or decreased mucin secretion such as Sjøgrens syndrome, dry mouth etc. Other disorders include dysregulation of selectin mediated leukocyte trafficking and would include but not be limited to disorders involving autoimmunity, arthritis, leukaemias, lymphomas, immunosuppression, sepsis, wound healing, acute and chronic inflammation, cell mediated immunity, and the like.

The agents identified by the methods described herein, may be useful in the prevention and treatment of tumors. Tumor metastasis may be inhibited or prevented by inhibiting the adhesion of circulating cancer cells. The substances, compounds, etc. of the invention may be especially useful in the treatment of various forms of neoplasia such as leukaemias, lymphomas, melanomas, adenomas, sarcomas, and carcinomas of solid tissues in patients. In particular the composition may be used for treating malignant melanoma, pancreatic cancer, cervico-uterine cancer, cancer of the liver, kidney, stomach, lung, rectum, breast, bowel, gastric, thyroid, neck, cervix, salivary gland, bile duct, pelvis, mediastinum, urethra, bronchogenic, bladder, esophagus and colon, and Kaposis Sarcoma which is a form of cancer associated with HIV-infected patients with Acquired Immune Deficiency Syndrome (AIDS). The substances etc. are particularly useful in the prevention and treatment of tumors of lining mucosa and glands and the metastases derived from these tumors.

Accordingly, the various agents may be formulated into pharmaceutical compositions for administration to subjects in a biologically compatible form suitable for administration in vivo. By biologically compatible form suitable for administration in vivo is meant a form of the agent to be administered in which any toxic effects are outweighed by the therapeutic effects. The agents may be administered to living organisms including humans, and animals. Administration of a therapeutically active amount of the pharmaceutical compositions of the present invention is defined as an amount effective, at dosages and for periods of time necessary to achieve the desired result. For example, a therapeutically active amount of an agent may vary according to factors such as the disease state, age, sex, and weight of the individual, and the ability of antibody to elicit a desired response in the individual. Dosage regima may be adjusted to provide the optimum therapeutic response. For example, several divided doses may be administered daily or the dose may be proportionally reduced as indicated by the exigencies of the therapeutic situation.

The active agent may be administered in a convenient manner such as by injection (subcutaneous, intravenous, etc.), oral administration, inhalation, transdermal application, or rectal administration. Depending on the route of administration, the active agent may be coated in a material to protect the agent from the action of enzymes, acids and other natural conditions that may inactivate it.

The compositions described herein can be prepared by methods known per se for the preparation of pharmaceutically acceptable compositions which can be administered to subjects, such that an effective quantity of the active agent is combined in a mixture with a pharmaceutically acceptable vehicle. Suitable vehicles are described, for example, in Remingtons Pharmaceutical Sciences (Remingtons Pharmaceutical Sciences, Mack Publishing Company, Easton, Pa., USA 1985). On this basis, the compositions include, albeit not exclusively, solutions of the agents in association with one or more pharmaceutically acceptable vehicles or diluents, and contained in buffered solutions with a suitable pH and iso-osmotic with the physiological fluids.

After pharmaceutical compositions have been prepared, they can be placed in an appropriate container and labelled for treatment of an indicated condition. For administration of an inhibitor of a polypeptide GalNAc-transferase, such labelling would include amount, frequency, and method of administration.

The use of inhibitors of the lectin domain mediated activities of the above mentioned polypeptide GalNAc-transferase isoforms and other isoforms allows for unique selective inhibition of these functions *in vitro* and *in vivo* in cells and organisms. This is desirable in manipulating the density of O-glycans, e.g. changing high density O-glycosylated tumor-associated MUC1 to low density normal MUC1 in cells. Further this is desirable in inhibiting any adhesive role the lectin domains may play in Golgi transport and intracellular sorting.

### 3. The MUC1 glycopeptide specificity of GalNAc-T4 is not directed by a specific glycoform.

The GalNAc-T4 isoform displays enzyme activity which, in addition to showing activity with some peptide substrates, exhibits unique activity with glycopeptides where prior glycosylation is a prerequisite for activity (8). GalNAc-T4 is unique in that it is the only GalNAc-transferase isoform identified so far that can complete the O-glycan attachment to all five acceptor sites in the 20 amino acid tandem repeat sequence (HGVTSAPDTRPAPGSTAPPA) of the breast cancer associated mucin, MUC1. GalNAc-T4 transfers GalNAc to at least two sites not used by other GalNAc-transferase isoforms on the GalNAc₄TAP24 glycopeptide (TAPPAHGVTSAPDTRPAPGSTAPP, GalNAc attachment sites underlined) (8). An activity such as that exhibited by GalNAc-T4 appears to be required for production of the glycoform of MUC1 expressed by cancer cells where all potential sites are glycosylated (10). In order to analyse activity of GalNAc-T4 with MUC1 derived GalNAc-peptides in detail different glycoforms of 24/25-mer peptides (TAP24/25) by using different GalNAc-transferase isoforms to catalyse glycosylation of selected sites in combination with valine substitutions of acceptor sites were prepared. Surprisingly, analysis of the substrate specificity of GalNAc-T4 with different glycoforms of MUC1 revealed that GalNAc-T4 did not show a requirement for any single site of GalNAc attachment (Fig. 1). By the contrary, there was only a requirement for at least one of the three sites to be glycosylated. Thus, substitution of any one of the sites glycosylated in the GalNAc₄TAP24/25 glycopeptide by valine did not affect activation of GalNAc-T4 activity for glycopeptides. Catalytic activity with certain sites was affected by site specific modifications, in particular glycosylation of S in -VTSA- or - GSTA- was influenced by glycosylation at adjacent and distant sites. This result suggested that a unique and novel triggering event of GalNAc-T4 activity existed in the presence of the glycosylated MUCl substrate. This activity could not be ascribed to simple conformational changes in the acceptor substrate induced by the glycosylation. This surprising finding led us to hypothesise that a triggering event that was independent of the general catalytic activity of the enzyme led to acquisition of specificity for GalNAc-glycopeptides.

### 4. The lectin domain of GalNAc-T4 selectively directs its MUC1 glycopeptide specificity

One potential candidate for such a triggering event of glycopeptide activity was the putative lectin domain, which was previously shown by mutational analysis to have no significant affect on the activity of the GalNAc-Tl isoform (3). Since GalNAc-T4 exhibits both glycosylation independent and glycosylation dependent activities, it offers a model system to analyse the different specificities as separate functions. Hagen *et al*. (3) originally demonstrated that critical substitutions in the lectin domain of GalNAc-T1 have little affect on catalytic activity (reduction by 10-50%) with peptide substrates, while substitutions in the catalytic domain destroyed activity (Fig. 2, Panel A). It was predicted that mutation of an aspartate residue adjacent to a conserved CLD motif in the lectin domain to histidine (D444H in GalNAc-T1 corresponding to D459H in GalNAc-T4) would destroy putative lectin function based on analysis of ricin (29), but mutation of this residue (D444H) in GalNAc-T1 only appeared to reduce activity by approximately 50 %. To test if the lectin domain influenced glycopeptide specificity of GalNAc-T4, recombinant secreted forms of GalNAc-T4^{459D} and -T4^{459H} were prepared. GalNAc-T4^{459D} and -T4^{459H} exhibited essentially the same specific activity with several unglycosylated peptides, in agreement with the results obtained for GalNAc-Tl (3) (illustrated for a PSGL-1 substrate in Fig. 2, Panel B). In contrast, the glycopeptide specificity of mutant GalNAc-T4^{459H} was selectively affected by the introduced mutations. Glycopeptides derived from tandem repeats of MUC1, MUC2 and MUC5AC (7) were virtually inactive as substrates, as is illustrated in Figure 2 (Panel C), which depicts assays with a GalNAc₃TAP25V21 glycopeptide. Essentially identical results were observed with unsubstituted TAP24 and GalNAc₄TAP24 glycopeptide. These results surprisingly demonstrates that the lectin domain is required for the glycopeptide specificity of enzyme activity, but not for activity with naked peptide substrates. This shows that the lectin domain triggers the catalytic domain of GalNAc-T4 to act on GalNAc-glycopeptide substrates by an as yet unknown mechanism. Furthermore, it demonstrates that the basic catalytic function and the triggering event are independent properties associated with distinct domains of GalNAc-transferases.

### 5. The lectin domain of GalNAc-T4 functions as a lectin and has selective specificity for GalNAc

In order to determine if actual carbohydrate binding contributed to the function of the lectin domain, we analysed whether triggering of glycopeptide specificity could be blocked by specific carbohydrates in solution. We could not detect direct binding of GalNAc-T2 and -T4 to free GalNAc using conventional binding assays presumably due to low affinity. More sensitive analyses will be required to demonstrate binding. However, as shown in Figure 3 (Panel A) the glycosylation dependent specificity of GalNAc-T4 was almost completely inhibited by incubation with 0.23 M free GalNAc, whereas other sugars, Gal, GlcNAc, or Fuc, failed to show significant inhibition. Assays with 50 mM sugars gave the same pattern, but with less (approximately 50 %) inhibition by GalNAc (not shown). Furthermore, similar inhibition was found with 10 mM α-D-GalNAc-1-benzyl, whereas αGlcNAc-benzyl did not inhibit catalytic activity. None of the sugars had significant affects on the glycosylation independent activities of GalNAc-T4^{459D} or -T4^{459H}, when assayed with naked peptides (Fig 3, Panel B). This demonstrates that the lectin domain of GalNAc-T4 must bind to GalNAc and contributes to the ability of GalNAc T4 to catalyse glycosylation of glycopeptides. It further demonstrates examples of inhibitors that selectively block the GalNAc-peptide substrate specificity of GalNAc-T4. The finding that neither Gal nor Galβ1-3GalNAcα1-benzyl produced significant inhibition compared to GalNAc suggests that the second step of O-glycosylation (extension of the oligosaccharide side chains), which is catalysed by the β3galactosyltransferase forming the core 1 structure Galβ1-3GalNAcα1-O-Ser/Thr, may block the functional activity of the lectin domain of GalNAc-T4. Thus, once the O-glycan processing step involving elongation to the core 1 structure is accomplished, GalNAc-T4 would not be capable of catalysing glycosylation of glycopeptides. This suggests that O-glycan elongation/branching and O-glycan density may be regulated by competition among GalNAc-transferases (lectin domain) and the glycosyltransferases involved in O-glycan extension, especially the core 1 synthase β3Gal-transferase.

### 6. The lectin domain of GalNAc-T7 functions as a lectin and has selective specificity for GalNAc and Galβ1-3GalNAc

GalNAc-T7 exhibits exclusive glycopeptide specificity and no unsubstituted acceptor peptide substrates have been identified thus far (7). GalNAc-T7 has a different glycopeptide substrate specificity than GalNAc-T4 and does not function with MUC1 derived glycopeptides. The best substrate identified to date is derived from the tandem repeat region of rat submaxillary gland mucin (30). The activity of GalNAc-T7 with GalNAc₂₋₃EA2 was significantly inhibited by benzyl-αGalNAc, benzyl-βGal, and the Galβ1-3GalNAcα1-benzyl disaccharide core 1 structure at 5 mM concentrations (Table I).

**Table I. Inhibition of GalNAc-T7 activity with GalNAc₂₋₃EA2 substrate**

| Activity (nmol/min/ml) in the presence of inhibitors (5 mM) | | | | |
|---|---|---|---|---|
| None | bz-αMan | bz-βGal | bz-αGalNAc | Galβ1-3GaINAcα1-bz |
| 6.8 | 6.7 | 4.7 | 5.4 | 4.5 |

### 7. The lectin domain of GalNAc-T2 is functional and has selective specificity for GalNAc and the MUC2 and MUC5AC tandem repeat peptides

GalNAc-T2 exhibits galactosyltransferase activity in the presence of the Muc2 acceptor substrate (28). Furthermore, testing a panel of peptide substrates it was found that GalNAc-T2 also utilized Muc7 and to lesser degree the EA2 peptide in the presence of UDP-Gal (GalNAc-T2 activity with UDP-Gal: Muc2, 90 nmol/min/ml; Muc7, 13 nmol/min/ml; EA2, 1.5 nmol/min/ml). The galactosyltransferase activity with Muc2 substrate was selectively inhibited by GalNAc and not other sugars.

**Table II. Inhibition of GalNAc-T2 activities with Muc2 acceptor substrate**

| Donor Substrate | Activity (nmol/min/ml) in the presence of inhibitors (230 mM) | | | | |
|---|---|---|---|---|---|
| | None | GalNAc | GlcNAc | Gal | Fuc |
| UDP-GalNAc | 340 | 300 | 310 | 300 | 370 |
| UDP-Gal | 90 | 24 | 68 | 89 | 89 |

Since the galactosyltransferase activity exhibited by GalNAc-T2 exhibits an entirely different acceptor substrate pattern than the N-acetylgalactosaminyltransferase activity, it is concluded that the lectin domain exhibits peptide binding specificity in addition to GalNAC. Hence, the mechanism of activation resemble that of the glycopeptide specificity of GalNAc-T4 only the trigger is a peptide sequence motif comprised in the Muc2 and Muc7 peptide sequences. Inhibitors to the lectin domain of GalNAc-T2 will block its ability to bind nascent unglycosylated MUC2 mucin polypeptides and hence affect a potential chaperone effect of GalNAc-T2 in Golgi.

### 8. The lectin domain of GalNAc-T3 is functional and has selective specificity for GalNAc and the MUC5AC and rat submaxillary tandem repeat peptides

GalNAc-T3 was found also to exhibit galactosyltransferase activity but only in the presence of the EA2 acceptor substrate (GalNAc-T2 activity with UDP-Gal: Muc2, 0 nmol/min/ml; Muc7, 0.1 nmol/min/ml; EA2, 6.8 nmol/min/ml). The galactosyltransferase activity with Muc2 substrate was selectively inhibited by GalNAc and not other sugars.

**Table III. Inhibition of GalNAc-T3 activities with EA2 acceptor substrate**

| Donor Substrate | Activity (nmol/min/ml) in the presence of inhibitors (230 mM) | | | | |
|---|---|---|---|---|---|
| | None | GalNAc | GlcNAc | Gal | Fuc |
| UDP-GalNAc | 34 | 35 | 32 | 33 | 34 |
| UDP-Gal | 6.3 | 2.5 | 7 | 7.3 | 6.5 |

The lectin domain of GalNAc-T3 resemble that of GalNAc-T2 in binding to peptide sequences although the sequence motif must be different and partly contained in the EA2 sequence.

These and other embodiments of the present invention are described in more detail below. The following examples are intended to further illustrate the invention without limiting its scope.

### EXAMPLES

### 1. Enzyme reaction conditions and substrates

Standard reaction mixtures (50 µl final volume) contained 25 mM cocadylate (pH 7.4), 10 mM MnCl₂, 0.25% Triton X-100, 200 µM UDP-[¹⁴C]-GalNAc (2,000 cpm/nmol) (Amer-sham), 200-500 µM acceptor peptides. Products were quantified by scintillation counting after chromatography on Dowex-1, octadecyl silica cartridges (Bakerbond), or HPLC (PC3.2/3 or mRPC C2/C18 SC2.1/10 Pharmacia, Smart System). Acceptor peptides included five variants of TAP25 (TAPPAHGV(T/V)SAPDTRPAPG(S/V)(T/V)APPA) and TAP24 (TAPPAHGVTSAPDTRPAPGSTAPP) derived from the human MUC1 tandem repeat (31); MUC2 (PTTTPISTTTMVTPTPTPTC) derived from human intestinal mucin MUC2 (32); MUC5AC (Ac-SAPTTSTTSAPT) derived from human respiratory gland mucin MUC5AC (33); MUC7 (Ac-CPPTPSATTPAPPSSSAPPETTAA) derived from human salivary gland mucin MUC7 (34); EA2 (PTTDSTTPAPTTK) derived from rat submandibular gland mucin (30); VTHPGY (Ac-PFVTHPGY) derived from human fibronectin (35); Zonadhesin (PTERTTTPTKRTTTPTIR) derived from human zonadhesin (36); OSM fragment (LSESTTQLPGGGPGCA) derived from ovine submaxillary mucin (37); hCG-β (PRFQDSSSSKAPPPLPSPSRLPG) derived from human chorionic gonadotropin β-subunit (38); MUC1b (RPAPGSTAPPA) derived from MUC1 and PSGL-1b (Ac-QATEYEYLDYDFLPETEPPEM) derived from the *N*-terminus of P-selectin ligand-1 (39). GalNAc-glycopeptides of MUC2, MUC5AC and MUC7 were produced using cold UDP-GalNAc and purified human recombinant GalNAc-T1 and -T2 (28). Different GalNAc-glycoforms of EA2 were produced by limiting the ratio of UDP-GalNAc to 2 moles, 3 moles, 4 moles or 5 moles per mole of acceptor peptide. Glycopeptides were purified on Supelclean LC-18 columns (1 ml, Supelco), and the number of GalNAc residues incorporated evaluated by MALDI-TOF mass spectrometry. The enzyme sources used were semipurified as previously described by successive sequential ion-exchange chromatographies on Amberlite (IRA95, Sigma) or DEAE Sephacel (Pharmacia), S-Sepharose Fast Flow (Pharmacia), and Mini-S^{™} (PC 3.2/3, Pharmacia) using the Smart System (Pharmacia) (28). Secreted GalNAc-T4 was obtained from a stably transfected CHO line (CHO/GalNA-T4/21A) (8) grown in roller bottles in HAMS F12 supplemented with 10 % Fetal Bovine Serum. The experiments illustrated in Fig. 1 was performed with recombinant secreted GalNAc-T4 obtained from a stably transfected CHO line (8). Experiments illustrated in Figs. 2 and 3 were performed with secreted GalNAc-T4 expressed in High Five cells grown in serum-free medium (8). Structural analysis of glycopeptides were performed by a combination of PFPA (pentafluoropropionic acid) hydrolysis and MALDI-TOF mass spectrometry as previously described (40). Secreted GalNAc-T7 was obtained from infected High Five^{™} cells grown in serum-free medium (Invitrogen) in upright roller bottles shaken 140 rpm in waterbaths at 27 °C. GalNAc-T7 was not purified by Mini-S as the yields from cationic chromatography were low due to its low pI (6.4).

### 2. Reaction kinetics monitored by Capillary Electrophoresis.

Reaction mixtures were modified to include 1.7 mM cold UDP-GalNAc, 25 µg acceptor peptides, and purified GalNAc-transferases in a final volume of 100 µl. The amount of GalNAc-transferase added was adjusted so that the reaction with the appropriate peptide was near completion in six hours. Reactions were incubated in the sample carousel of an Applied Biosystem model HT270 at 30°C as described previously (28). Electrophoretograms were produced every 60 min, and after six hours the reaction mixtures were separated by reverse phase HPLC for structural determination. HPLC was performed on a Brawnlee ODS column (2.1 mm x 30 mm, 5 µm particle size) (Applied Biosystems, Inc.) using a linear gradient (0-30%, 0.1% TFA/ 0.08% TFA, 90% acetonitrile, 30 min) delivered by an ABI 130A micro-bore HPLC system (Perkin Elmer Inc).

### 3. Structural analysis of reaction products

Glycopeptides were purified by HPLC and analysed by a combination of PFPA (pentafluoropropionic acid, Sigma) hydrolysis and MALDI-TOF mass spectrometry. Glycopeptides (50 pmol) were lyophilized in 500 µl Eppendorf vials and placed in a 22 ml glass vial with a mininert valve (Pierce, Rockford, IL). A solution of 100 µl 20% PFPA (aqueous) containing 500 µg DTT was added to the bottom of the glass vial, which was then flushed with argon. The vial was evacuated to 1 mbar, and placed in an oven at 90°C for 60 min. The hydrolyzed samples were centrifuged in a vacuum centrifuge for 15 min to remove remaining traces of acid. Lyophilized samples were reconstituted in 0.1% TFA to a concentration of 1 pmol/µl. Mass spectra were acquired on either Voyager-DE or Voyager-Elite mass spectrometers equipped with delayed extraction (Perseptive Biosystem Inc.). The matrix used was 2,5-dihydroxybenzoic acid (10 mg/ml, Hewlett-Packard) dissolved in a 2:1 mixture of 0.1% trifluoroacetic acid in 30 % aqueous acetonitrile (Rathburn Ltd.). Samples dissolved in 0.1% trifluoroacetic acid to a concentration of approximately 80 fmol to 1 pmol/ml were prepared for analysis by placing 1 µl of sample solution on a probe tip followed by 1µl of matrix. The hydrolyzed samples were prepared for MALDI analysis using nano-scale reversed-phase columns (Poros R3, PerSeptive Biosystem), according to previously described procedure (41). Samples were prepared by mixing 0.8 µl of total fraction volume 2 pmol of hydrolyzed glycopeptides and 0.4 µl of matrix solution. Mass spectra were acquired in reflector mode on a Voyager-Elite Biospectrometry Workstation (PerSeptive Biosystems Inc., Framingham, Ma, USA) equipped with delayed ion extraction technology. Data processing was performed using software packages Perseptive-Grams (Galactic Industries Corp.) and protein analysis software GPMAW
(htpp://www.welcome.to/gpmaw; Lighthouse data, Odense, Denmark)

### 4. Reaction kinetics monitored by mass spectrometry

MALDI-TOF time-course in terminal reactions were performed in reactions of 25 µl containing 2.5 nmol acceptor (glyco)peptide, 40 nmol UDP-GalNAc, and 0.4 µg GalNAc-T4. Sampling of reactions (1 µl) were purified by nano-scale reversed-phase chromatography (Poros R3, PerSeptive Biosystem) and applied directly to the probe with matrix (41). The amount of GalNAc-transferase added was adjusted so that the reaction with the appropriate peptide was near completion in six hours. Reactions were incubated at 37°C in a shaker bath. At times 0, 2 hours, and 16 hours a 1 µl aliquot was taken and purified. Mass spectra were acquired on either Voyager-DE mass spectrometer equipped with delayed extraction (Perseptive Biosystem Inc.). The matrix used was 2,5-dihydroxybenzoic acid (10 mg/ml, Hewlett-Packard) dissolved in a 2:1 mixture of 0.1% trifluoroacetic acid in 30 % aqueous acetonitrile (Rathburn Ltd.).

### 5. Construction, expression, purification, and analysis of a lectin domain mutant of GalNAc-T4

The mutant GalNAc-T4^{459H} was prepared by multiplex PCR using the GalNAc-T4-sol construct that encodes residues 32-578 inserted into pT7T3U19 (8). Primers EBHC332 (5'-GTAGAGGGATCTCGTCTGAATGTTTACATTATA-3' (mutation under-lined in bold)) and T7 (5'-TAATACGACTCACTATAGGG-3) were used in a standard reaction under the following cycling conditions; 95 °C 45 s, 51 °C 5 s, 72 °C 1 min. For 18 cycles using a Tc 2400 thermocycler (PE Biosystems, USA). The PCR product was digested with *Bst*YI gel purified. 5 ng hereof was mixed with 10 ng pAcGP67-GalNAc-T4sol (8), and the mixture used to prime a "shuffle PCR" reaction using primers T7/EBHC201 (5'AAGCGGGCACCATATGCTCG-3'), using standard conditions and the following cycling conditions 95 °C 45 s, 51 °C 5 s, 72 °C 1 min (5 cycles without primers, after which primers were added and the reactions was cycled for an additional 17 cycles). The generated PCR product was digested with *Hind*III and inserted into *Hind*III digested GalNAc-T4 pT7T3U19 construct described above. The mutated T4- construct was fully sequenced and the BamHI insert subcloned into pAcGP67.

Wild-type and mutant constructs expressed in insect cells were secreted in comparable yields, and the purified proteins migrated by SDS-PAGE identically. Quantification of purified proteins was done by Coomassie stained SDS-PAGE and titration of immunoreactivity with the monoclonal antibody, UH6(4G2) (8). GalNAc-T4^{459D} and -T4^{459H} were purified to 0.04 µg/µl and 0.1 µg/µl with specific activities of 0.197 U/mg and 0.24 U/mg with a MUC7 tandem repeat derived peptide (7), respectively. Wild-type GalNAc-T4 and mutant GalNAc-T4, GalNAc-T4^{459H}, were analysed with unglycosylated peptides (represented by PSGL-1) or GalNAc glycosylated glycopeptides (represented by GalNAc₃TAP25V21) in reactions of 25 µl containing 2.5 nmol acceptor (glyco)peptide, 40 nmol UDP-GalNAc, and 0.4 µg GalNAc-T4. Time-course assays were motioned by MALDI-TOF. Sampling of reactions (1 µl) were purified by nano-scale reversed-phase chromatography (Poros R3, PerSeptive Biosystem) and applied directly to the probe with matrix. Evaluation of inhibition of the glycopeptide specificity of wild-type GalNAc-T4 with free sugars was performed to establish if the lectin domain recognized carbohydrate. Analysis was performed as above with 0.23 M free GalNAc, Gal, GlcNAc, or Fuc, and the reaction was monitored by MALDI-TOF. Further, analysis was performed with 10 mM α-D-GalNAc-1-benzyl, αGlcNAc-benzyl, and fully occupied GalNAc-glycopeptide, GalNAc₆TAP25, at 5 mM.

### 6. Inhibition of the GalNAc-glycopeptide activity of GalNAc-T7

GalNAc-T7 activity was analysed with GalNAc glycosylated glycopeptides (represented by GalNAc₂₋₃EA2 (7)) in reactions of 25 µl containing 2.5 nmol acceptor (glyco)peptide, 40 nmol UDP-GalNAc, and purified GalNAc-T7. Assays were performed with 0.23 M free GalNAc, Gal, GlcNAc, or Fuc, and the reaction product quantified by Dowex-1 chromatography and scintillation counting. Further, analysis was performed with 10 mM α-D-GalNAc-1-benzyl, αGlcNAc-benzyl, and fully occupied GalNAc-glycopeptide, GalNAc₆TAP25, at 5 mM.

### 7. Inhibition of lectin domains of GalNAc-transferases, GalNAc-T2 and -T3, that do not exhibit glycopeptide specificities

GalNAc-T2 exhibits activity with UDP-Gal in the presence of the acceptor substrate Muc2 (28). Galactosyl transferring activities of GalNAc-T1, -T2, and -T3, were assayed with a panel of acceptor peptides in standard reaction mixtures containing 100 µM UDP-Gal instead of UDP-GalNAc. GalNAc-T2 showed activity with Muc2 as well as low activity with Muc7 and very low activity with EA2 acceptor substrates. GalNAc-T3 showed activity with EA2 and lower activity with Muc7, but no activity with other peptides tested. Since the activities with UDP-Gal do not correlate with the general acceptor substrate specificities of these GalNAc-transferase isoforms found with the UDP-GalNAc donor substrate, it was tested if the lectin domains were involved. This was done by analysing if free sugars could selectively inhibit the activities with UDP-Gal and not UDP-GalNAc. Assays were performed with 0.23 M free GalNAc, Gal, GlcNAc, or Fuc, and the reaction product quantified by Dowex-1 chromatography and scintillation counting. Further, analysis was performed with 10 mM α-D-GalNAc-1-benzyl, αGlcNAc-benzyl, and fully occupied GalNAc-glycopeptide, GalNAc₆TAP25, at 5 mM.

### REFERENCES

1. Paulson, J.C. and Colley, K.J. Glycosyltransferases. Structure, localization, and control of cell type-specific glycosylation. J.Biol.Chem., *264:* 17615-17618, 1989.
2. Hassan, H., Bennett, E.P., Mandel, U., Hollingsworth, M.A., and Clausen, H. Control of Mucin-Type O-Glycosylation: O-Glycan Occupancy is Directed by Substrate Specificities of Polypeptide GalNAc-Transferases. In: G.W. Hart and M. Fukuda (eds.), pp. 273-292, New York: Wiley-VCH. 2000.
3. Hagen, F.K., Hazes, B., Raffo, R., deSa, D., and Tabak, L.A. Structure-Function Analysis of the UDP-N-acetyl-D- galactosamine:Polypeptide N-acetylgalactosaminyltransferase. Essential residues lie in a predicted active site cleft resembling a lactose repressor fold. J. Biol. Chem., *274*: 6797-6803, 1999.
4. Hazes, B. The (QxW)3 domain: a flexible lectin scaffold. Protein Science, *5*: 1490-1501, 1996.
5. Imberty A., Piller V., Piller F., and Breton C. Fold recognition and molecular modeling of a lectin-like domain in UDP-GalNac: polypeptide N-acetylgalactosaminyltransferases. Protein Eng., 10: 1353-1356, 1997.
6. Breton, C. and Imberty, A. Structure/function studies of glycosyltransferases. Curr.Opin.Struct.Biol, 9: 563-571, 1999.
7. Bennett, E.P., Hassan, H., Hollingsworth, M.A., and Clausen, H. A novel human UDP-*N*-acetyl-D-Galactosamine:polypeptide *N*-acetylgalactosaminyltransferase, GalNAc-T7, with specificity for partial GalNAc-glycosylated acceptor substrates. FEBS Letters, *460:* 226-230, 1999.
8. Bennett, E.P., Hassan, H., Mandel, U., Mirgorodskaya, E., Roepstorff, P., Burchell, J., Taylor-Papadamitriou, J., Hollingsworth, M.A., Merkx, G., Geurts van Kessel, A., Eiberg, H., Steffensen, R., and Clausen, H. Cloning of a human UDP-*N*-acetyl- -D-galactosamine: polypeptide *N*-acetylgalactosaminyltransferase that complements other GalNAc-transferases in complete *O-*glycosylation of the MUC1 tandem repeat. J. Biol. Chem., *273*: 30472-30481, 1998.
9. Ten Hagen, K.G., Tetaert, D., Hagen, F.K., Richet, C., Beres, T.M., Gagnon, J., Balys, M.M., VanWuyckhuyse, B., Bedi, G.S., Degand, P., and Tabak, L.A. Characterization of a UDP-GalNAc:polypeptide N-acetylgalactosaminyltransferase that displays glycopeptide N-acetylgalactosaminyltransferase activity. J. Biol. Chem., *274*: 27867-27874, 1999.
10. Muller, S., Alving, K., Peter-Katalinic, J., Zachara, N., Gooley, A.A., and Hanisch, F.G. High density O-glycosylation on tandem repeat peptide from secretory MUC1 of T47D breast cancer cells. J. Biol. Chem., *274*, 1999.
11. Muller, S., Goletz, S., Packer, N., Gooley, A.A., Lawson, A.M., and Hanisch, F.G. Localization of 0-glycosylation sites on glycopeptide fragments from lactation-associated MUC1. J. Biol. Chem., *272*: 24780-24793, 1997.
12. Jentoft, N. Why are proteins O-glycosylated?. Trends Biochem. Sci., *15*: 291-294, 1990
13. Tabak, L.A. In defense of the oral cavity: structure, biosynthesis, and function of salivary mucins. Annual Review of Physiology, *57*: 547-564, 1995.
14. Van den Steen, P., Rudd, P.M., Dwek, R.A., and Opdenakker, G. Concepts and principles of O-linked glycosylation. Crit.Rev.Biochem Mol.Biol., *33*: 151-208, 1998.
15. Taylor-Papadimitriou, J. and Epenetos, A.A. Exploiting altered glycosylation patterns in cancer: progress and challenges in diagnosis and therapy. Trends In Biotechnology, *12*: 227-233, 1994.
16. Taylor-Papadimitriou, J. and Finn, O.J. Biology, biochemistry and immunology of carcinoma-associated mucins. Immunology Today, *18*: 105-107, 1997.
17. Scharfman, A., Lamblin, G., and Roussel, P. Interactions between human respiratory mucins and pathogens. Biochemical Society Transactions, 23: 836-839, 1995.
18. Rose, M.C. Mucins: structure, function, and role in pulmonary diseases. Am. J. Physiol. 1992. Oct., *263*: L413-L429
19. Thomsson, K.A., Carlstedt, I., Karlsson, N.G., Karlsson, H., and Hansson, G.C. Different O-glycosylation of respiratory mucin glycopeptides from a patient with cystic fibrosis. Glycoconj. J., *15*: 823-833, 1998
20. Kuan, S.F., Byrd, J.C., Basbaum, C., and Kim, Y.S. Inhibition of mucin glycosylation by aryl-N-acetyl-alpha-galactosaminides in human colon cancer cells. J. Biol. Chem., *264*: 19271-19277, 1989
21. Huet, G., Hennebicq-Reig, S., de Bolos, C., Ulloa, F., Lesuffleur, T., Barbat, A., Carriere, V., Kim, I., Real, F.X., Delannoy, P., and Zweibaum, A. GalNAc-alpha-O-benzyl inhibits NeuAcalpha2-3 glycosylation and blocks the intracellular transport of apical glycoproteins and mucus in differentiated HT-29 cells. J. Cell Biol., *141*: 1311-1322, 1998.
22. Byrd, J.C., Dahiya, R., Huang, J., and Kim, Y.S. Inhibition of mucin synthesis by benzyl-alpha-GalNAc in KATO III gastric cancer and Caco-2 colon cancer cells. Eur. J. Cancer, *31A*: 1498-1505, 1995.
23. Alfalah, M., Jacob, R., Preuss, U., Zimmer, K.P., Naim, H., and Naim, H.Y. O-linked glycans mediate apical sorting of human intestinal sucrase-isomaltase through association with lipid rafts. Curr.Biol, *9*: 593-596, 1999.
24. Yeaman, C., Le Gall, A.H., Baldwin, A.N., Monlauzeur, L., Le Bivic, A., and Rodriguez-Boulan, E. The O-glycosylated stalk domain is required for apical sorting of neurotrophin receptors in polarized MDCK cells. J. Cell Biol., *139*: 929-940, 1997.
25. Ulloa, F., Franci, C., and Real, F.X. GalNAc-a-O-Benzyl inhibits sialylation of de novo synthesized apical, but not basolateral, sialoglycoproteins and blocks lysosomal enzyme processing in a post-TGN compartment. J. Biol. Chem. 2000. Prepublished Apr.5.
26. White, T., Bennett, E.P., Takio, K., Sorensen, T., Bonding, N., and Clausen, H. Purification and cDNA cloning of a human UDP-N-acetyl-alpha-D- galactosamine:polypeptide N-acetylgalactosaminyltransferase. J. Biol. Chem., *270*: 24156-24165, 1995.
27. Bennett, E.P., Hassan, H., and Clausen, H. cDNA cloning and expression of a novel human UDP-N-acetyl-alpha-D-galactosamine. Polypeptide N-acetylgalactosaminyltransferase, GalNAc-t3. J. Biol. Chem., *271*: 17006-17012, 1996.
28. Wandall, H.H., Hassan, H., Mirgorodskaya, E., Kristensen, A.K., Roepstorff, P., Bennett, E.P., Nielsen, P.A., Hollingsworth, M.A., Burchell, J., Taylor-Papadimitriou, J., and Clausen, H. Substrate specificities of three members of the human UDP-N-acetyl-alpha-D-galactosamine:Polypeptide N-acetylgalactosaminyltransferase family, GalNAc-T1, -T2, and -T3. J. Biol. Chem., *272*: 23503-23514, 1997.
29. Day, P.J., Ernst, S.R., Frankel, A.E., Monzingo, A.F., Pascal, J.M., Molina-Svinth, M.C., and Rober-tus, J.D. Structure and activity of an active site substitution of ricin A chain. Biochemistry, *35*: 11098-11103, 1996.
30. Albone, E.F., Hagen, F.K., VanWuyckhuyse, B.C., and Tabak, L.A. Molecular cloning of a rat submandibular gland apomucin. J. Biol. Chem. *269*: 16845-16852, 1994.
31. Gendler, S.J., Spicer, A.P., Lalani, E.N., Duhig, T., Peat, N., Burchell, J., Pemberton, L., Boshell, M., and Taylor-Papadimitriou, J. Structure and biology of a carcinoma-associated mucin, MUC1. American Review of Respiratory Disease *144*: S42-7, 1991.
32. Gum, J.R., Byrd, J.C., Hicks, J.W., Toribara, N.W., Lamport, D.T., and Kim, Y.S. Molecular cloning of human intestinal mucin cDNAs. Sequence analysis and evidence for genetic polymorphism. J. Biol. Chem. *264*: 6480-6487, 1989.
33. Guyonnet, D.V., Audie, J.P., Debailleul, V., Laine, A., Buisine, M.P., Galiegue-Zouitina, S., Pigny, P., Degand, P., Aubert, J.P., and Porchet, N. Characterization of the human mucin gene MUC5AC: a consensus cysteine- rich domain for 11p15 mucin genes? Biochem. J., *305*: 211-219, 1995
34. Bobek, L.A., Tsai, H., Biesbrock, A.R., and Levine, M.J. Molecular cloning, sequence, and specificity of expression of the gene encoding the low molecular weight human salivary mucin (MUC7). J. Biol. Chem. *268*: 20563-20569, 1993.
35. Matsuura, H., Greene, T., and Hakomori, S. An alpha-N-acetylgalactosaminylation at the threonine residue of a defined peptide sequence creates the oncofetal peptide epitope in human fibronectin. J. Biol. Chem. *264*: 10472-10476, 1989.
36. Hardy, D.M. and Garbers, D.L. A sperm membrane protein that binds in a species-specific manner to the egg extracellular matrix is homologous to von Wille-brand factor. J. Biol. Chem. *270* : 26025-26028, 1995.
37. Hill, H.D.J., Schwyzer, M., Steinman, H.M., and Hill, R.L. Ovine submaxillary mucin. Primary structure and peptide substrates of UDP-N-acetylgalactosamine:mucin transferase. J. Biol. Chem. *252*: 3799-3804, 1977
38. Birken, S. and Canfield, R.E. Isolation and amino acid sequence of COOH-terminal fragments from the beta subunit of human choriogonadotropin. J. Biol. Chem. *252*: 5386-5392, 1977
39. Sako, D., Comess, K.M., Barone, K.M., Camphausen, R.T., Cumming, D.A., and Shaw, G.D. A sulfated peptide segment at the amino terminus of PSGL-1 is critical for P-selectin binding. Cell *83* : 323-331, 1995.
40. Mirgorodskaya, E., Hassan, H., Wandall, H.H., Clausen, H., and Roepstorff, P. Partial Vapor-Phase Hydrolysis of Peptide Bonds: A Method for Mass Spectrometric Determination of O-Glycosylated Sites in Glycopeptides. Anal. Biochem. *269*: 54-65, 1999.
41. Gobom, J., Nordhoff, E., Mirgorodskaya, E., Ekman, R., and Roepstorff, P. Sample purification and preparation technique based on nano-scale reversed-phase columns for the sensitive analysis of complex peptide mixtures by matrix-assisted laser desorption/ionization mass spectrometry. J. Mass Spectrom. *34*: 105-116, 1999.

## Claims

1. A method of screening one or more test substances for the ability to modulate enzymatic activity of polypeptide GalNAc-transferases mediated by lectin domains therein in a cell-free or cell-based assay, which comprises:
(i) contacting a polypeptide GalNAc-transferase, or a cell that recombinantly expresses a polypeptide GalNAc-transferase, with one or more test substances, which are selected from the group consisting of carbohydrates, glycopeptides, glycoconjugates and portions and fragments thereof and are inactive as acceptor substrates for glycosyltransferases, under assay conditions suitable for the detection of said enzymatic activity; and
(ii) measuring whether said enzymatic activity is thereby modulated by one or more of the test substances in a way similar to the enzyme having an inactivating mutation in its lectin domain; or
(iii) measuring whether said enzymatic activity is thereby modulated by one or more of the test substances in a way similar to the enzyme functioning in the presence of a GalNAc residue or α-D-GalNAc-1-benzyl.

2. The method of claim 1 wherein the polypeptide GalNAc-transferase is selected from the group consisting of GalNAc-T2, GalNAc-T3, GalNAc-T4 and GalNAc-T7.

3. The method of claim 1, wherein the polypeptide GalNAc-transferase is GalNAc-T3.

4. The method of claim 1, wherein the polypeptide GalNAc-transferase is GalNAc-T4.

5. The method of claim 1, wherein the polypeptide GalNAc-transferase is GalNAc-T2.

6. The method of claim 1, wherein the polypeptide GalNAc-transferase is GalNAc-T7.

7. The method according to any one of claims 1-6, wherein said assay conditions of step (i) include a donor substrate that is UDP-*N*-acetylgalactosamine or UDP-Gal.

8. The method according to any one of claims 1-7, wherein said assay conditions of step (i) include an acceptor substrate that is a GalNAc glycosylated glycopeptide.

9. The method according to claim 8, wherein said acceptor substrate is GalNAc₃TAP25V21 or GalNAc₂₋₃EA2.

## Patentansprüche

1. Verfahren zum Testen einer oder mehrerer Testsubstanzen auf die Eigenschaft zur Modulation der enzymatischen Aktivität von Polypeptid-GalNAc-Transferasen, vermittelt durch deren Lectin-Domänen, in einem zellfreien oder zellbasierten Test, umfassend:
(i) in Kontakt bringen von einer Polypeptid-GalNAc-Transferase oder einer Zelle, die eine Polypeptid-GalNAc-Transferase rekombinant exprimiert, mit einer oder mehreren Testsubstanzen, die ausgewählt werden aus der Gruppe bestehend aus Kohlenhydraten, Glycopeptiden, Glycokonjugaten und Bereichen und Fragmenten davon und die als Akzeptor-Substrate für Glycosyltransferasen inaktiv sind, unter Testbedingungen, die zur Detektion der enzymatischen Aktivität geeignet sind; und
(ii) messen, ob die enzymatische Aktivität dabei durch eine oder mehrere der Testsubstanzen auf analoge Weise wie beim Enzym mit einer inaktivierenden Mutation in seiner Lectin-Domäne beeinflusst wird; oder
(iii) messen, ob die enzymatische Aktivität dabei durch eine oder mehrere der Testsubstanzen auf analoge Weise wie beim Enzym, das in der Gegenwart eines GalNAc-Rest oder α-D-GalNAc-1-benzyl reagiert, beeinflusst wird.

2. Verfahren gemäss Anspruch 1, wobei die Polypeptid-GalNAc-Transferase aus der Gruppe bestehend aus GalNAc-T2, GalNAc-T3, GalNAc-T4 und GalNAc-T7 ausgewählt wird.

3. Verfahren gemäss Anspruch 1, wobei die Polypeptid-GalNAc-Transferase GalNAc-T3 ist.

4. Verfahren gemäss Anspruch 1, wobei die Polypeptid-GalNAc-Transferase GalNAc-T4 ist.

5. Verfahren gemäss Anspruch 1, wobei die Polypeptid-GalNAc-Transferase GalNAc-T2 ist.

6. Verfahren gemäss Anspruch 1, wobei die Polypeptid-GalNAc-Transferase GalNAc-T7 ist.

7. Verfahren nach irgend einem der Ansprüche 1-6, wobei die Testbedingen von Schritt (i) ein Donor-Substrat, das ein UDP-*N*-acetylgalactosamin oder UDP-Gal ist, einschliessen.

8. Verfahren nach irgend einem der Anprüche 1-7, wobei die Testbedingungen von Schritt (i) ein Akzeptor-Substrat, das ein GalNAc glykosyliertes Glykopeptid ist, einschliessen.

9. Verfahren nach Anspruch 8, wobei das Akzeptor-Substrat GalNAc₃TAP25V21 oder GalNAc₂₋₃EA2 ist

## Revendications

1. Procédé de criblage d'une ou de plusieurs substances à l'essai pour l'aptitude à moduler l'activité enzymatique de polypeptide-GalNAc-transférases médiée par les domaines lectine à l'intérieur de celles-ci, dans un dosage sans cellules ou à base de cellules, qui comprend les étapes consistant à :
(i) mettre en contact une polypeptide- GalNAc-transférase, ou une cellule qui exprime de façon recombinante une polypeptide-GalNAc-transférase, avec une ou plusieurs substances à l'essai qui sont choisies dans le groupe constitué des hydrates de carbone, des glycopeptides, des glycoconjugués et de portions et fragments de ceux-ci et qui sont inactives en tant que substrats accepteurs pour les glycosyltransférases, dans des conditions de dosage appropriées à la détection de ladite activité enzymatique ; et
(ii) mesurer si ladite activité enzymatique est modulée de ce fait par l'une ou plusieurs des substances à l'essai d'une manière similaire à l'enzyme ayant une mutation inactivante dans son domaine lectine ; ou
(iii) mesurer si ladite activité enzymatique est modulée de ce fait par l'une ou plusieurs des substances à l'essai d'une manière similaire à l'enzyme fonctionnant en présence d'un résidu GalNAc ou d'α-D-GalNAc-1-benzyle.

2. Procédé selon la revendication 1, **caractérisé en ce que** la polypeptide-GalNAc-transférase est choisie dans le groupe constitué de GalNac-T2, de GalNAc-T3, de GalNAc-T4 et de GalNAc-T7.

3. Procédé selon la revendication 1, **caractérisé en ce que** la polypeptide-GalNAc-transférase est GalNAc-T3.

4. Procédé selon la revendication 1, **caractérisé en ce que** la polypeptide-GalNAc-transférase est GalNAc-T4.

5. Procédé selon la revendication 1, **caractérisé en ce que** la polypeptide-GalNAc-transférase est GalNAc-T2.

6. Procédé selon la revendication 1, **caractérisé en ce que** la polypeptide-GalNAc-transférase est GalNAc-T7.

7. Procédé selon l'une quelconque des revendications 1 à 6, **caractérisé en ce que** lesdites conditions de dosage de l'étape (i) incluent un substrat donneur qui est l'UDP-*N*-acétylgalactosamine ou l'UDP-Gal.

8. Procédé selon l'une quelconque des revendications 1 à 7, **caractérisé en ce que** lesdites conditions de dosage de l'étape (i) incluent un substrat accepteur qui est un glycopeptide glycosylé par GalNAc.

9. Procédé selon la revendication 8, **caractérisé en ce que** ledit substrat accepteur est GalNAc₃TAP25V21 ou GalNAc₂₋₃EA2.


### SEQUENCE LISTING

<110> HASSAN, Helle
   BENNETT, Eric Paul
   CLAUSEN, Henrik
<120> Methods of modulating functions of polypeptide GalNAc-transferases and of screening test substances to find agents herefor, etc.
<130> P200000587 WO JNY
<140> PCT/DK01/00328
   <141> 2001-10-05
<150> US 60/203,331
   <151> 2000-10-11
<160> 3
<170> PatentIn version 3.1
<210> 1
   <211> 20
   <212> DNA
   <213> Artificial
<220>
   <221> primer_bind
   <222> (1)..(20)
   <223> Primer sequence EBHC201
<400> 1
   aagcgggcac catatgctcg 20
<210> 2
   <211> 33
   <212> DNA
   <213> Artificial
<220>
   <221> primer_bind
   <222> (1)..(33)
   <223> Primer sequence EBCH332
<400> 2
   gtagagggat ctcgtctgaa tgtttacatt ata 33
<210> 3
   <211> 20
   <212> DNA
   <213> Artificial
<220>
   <221> primer_bind
   <222> (1)..(20)
   <223> Primer sequence T7
<400> 3
   taatacgact cactataggg 20